# EUROPÄISCHE PATENTANMELDUNG

(11) **EP 0 979 810 A1**
(43) Veröffentlichungstag der Anmeldung: **16.02.2000**
(21) Anmeldenummer: 99114188.8
(22) Anmeldetag: 23.07.1999
(51) Int. Cl.: C07C 45/39, C07C 49/443, B01J 27/188, B01J 27/199

(54) **Verfahren zur katalytischen Oxidation von 1,5-Dialkylbicyclo(3.2.1)octan-8-ol zu 1,5-Diaklylbicyclo(3.2.1)octan-8-on**

(30) Priorität: 05.08.1998 DE 19835323
(71) Anmelder: Aventis Research & Technologies GmbH & Co. KG, 65926 Frankfurt am Main (DE)
(72) Erfinder: Hagemeyer, Alfred, Dr., 48431 Rheine (DE); Schulz, Rolf Peter, Dr., 46539 Dinslaken (DE); Werner,Harald, Dr., 61350 Bad Homburg (DE); Dingerdissen, Uwe, Dr., 64342 Seeheim-Jugenheim (DE); Kühlein, Klaus, Prof. Dr., 65779 Kelkheim (DE); Alexander, Heinz, 65929 Frankfurt (DE)

(57) **Zusammenfassung**

Verfahren zur Herstellung von Bicyclooctanon insbesondere 1,5-Dialkylbicyclo(3.2.1)octan-8-on durch Umsetzung von Bicyclooctanol insbesondere Dialkylbicyclo(3.2.1)octan-8-ol mit
a. einem auf einem Aktivkohleträger aufgebrachten Katalysator enthaltend Heteropolyoxometallatanionen von Vanadium, Molybdän und Phosphor sowie ein entsprechendes Alkali-, Erdalkali- und/oder Ammonium-Gegenion und
b. einem gasförmigen, sauerstoffhaltigen Oxidationsmittel

## Beschreibung

Die vorliegende Erfindung betrifft ein katalytisches Verfahren zur Selektivoxidation von Bicyclooctanol zu Bicyclooctanon mit einem aufAktivkohle geträgerten Heteropolymetallatanion als Katalysator. Insbesondere betrifft die Erfindung die Oxidation von 1,5-Dimethylbicyclo(3.2.1)octan-8-ol zum Keton mit Sauerstoff, Sauerstoff enthaltenden Gasen oder Luft.

Für die Oxidation sekundärer Alkohole zu Ketonen sind verschiedene Verfahren bekannt. Diese konventionellen Oxidationsverfahren sind jedoch mit zahlreichen Nachteilen behaftet, wie z.B.
- der Einsatz von teueren Oxidationsmitteln in stöchiometrischen Mengen oder sogar im Überschuß,
- der Abtrennung und Entsorgung von verbrauchtem und unverbrauchtem Oxidationsmittel,
- Salzanfall, was Entsorgungskosten nach sich zieht und die Produktabtrennung erschwert,
- hohe Drucke
- mangelnde Selektivität
- unvollständige Umsätze.
Bekannte etablierte Verfahren sind z.B. die Oppenauer-Oxidation mit Al-Systemen oder die H₂O₂-Oxidation mit Ti, Mo, V-Systemen oder Transfer-(De-)Hydrierungen.

Daher sind die konventionellen Verfahren oft kosten- und energieintensiv und mit erheblichen Stofftrennungsproblemen bei der Isolierung des Produktes aus einer komplexen Reaktionsmischung belastet. Die umweltgerechte Entsorgung von Oxidationsmitteln und Hilfschemikalien bereitet oft Probleme.

Es war eine Aufgabe der vorliegenden Erfindung, ein kostengünstiges und umweltfreundliches Verfahren zur Selektivoxidation von Bicyclooctanolen zu den Ketonen bereitzustellen, das die Nachteile der konventionellen Oxidationen vermeidet.

Es wurde nun gefunden, daß mit einem aufAktivkohle geträgerten Katalysator enthaltend Heteropolymetallatanion ein Oxidationsverfahren möglich ist,
das unter milden Reaktionsbedingungen, sogar drucklos,
mit billigen Oxidationsmitteln wie Sauerstoff enthaltenden Gasen, sogar Luft,
ohne Salzanfall,
hohe Selektivitäten,
hohe Umsätze, ja sogar Vollumsatz,
erzielt.

Die vorliegende Erfindung betrifft somit Verfahren zur Herstellung von Bicyclooctanon, insbesondere 1,5-Dialkylbicyclo(3.2.1.)octan-8-on, durch Umsetzung von Bicyclooctanol, insbesondere 1,5-Dialkylbicyclo(3.2.1.)octan-8-ol , mit a. einem auf einem Aktivkohleträger aufgebrachten Katalysator enthaltend Heteropolyoxometallatanionen von Vanadium, Molybdän und Phosphor sowie ein entsprechendes Alkali-, Erdalkali- und/oder Ammonium-Gegenion und b. einem gasförmigen, sauerstoffhaltigen Oxidationsmittel.

Alkyl steht unabhängig voneinander bevorzugt für einen verzweigten oder unverzweigten H, Methyl, Ethyl, Propyl, Butyl, Pentyl, Hexyl oder Cyclohexylrest.

Als Oxidationsmittel wird Sauerstoff, Sauerstoff enthaltende Gase oder Luft verwendet.

Die erfindungsgemäße Oxidation kann im Temperaturbereich von 100 - 300°C, vorzugsweise 120 bis 250°C und Druckbereich von 1 - 30 bar, vorzugsweise 1 bis 25 bar, in verschiedenen Lösungsmitteln oder in Substanz betrieben werden. Im Falle der Dimethylverbindungen wird bevorzugt bei 140 - 250°C und bei 1 - 20 bar gearbeitet. Als Oxidationsmittel kommen reiner oder verdünnter Sauerstoff, insbesondere Luft oder Magerluft, zur Anwendung.
Eine besondere Ausführungsform der vorliegenden Erfindung arbeitet drucklos bzw. unter Normaldruck, wobei das Sauerstoff enthaltende Gas oder Luft unter Normaldruck (bzw. dem sich ergebenden Druckabfall über den Reaktor) direkt z.B. mittels einer Eindüsvorrichtung in die flüssige Reaktionsmischung kontinuierlich oder batchweise eingeleitet wird.

Bei einer Reaktionsführung mit Vollumsatz wird das Problem der Eduktabtrennung und dessen Rückführung aus dem Reaktionsgemisch vermieden und daher die Stofftrennung enorm vereinfacht.
Für den Katalysator steht eine einfache, reproduzierbare und hochskalierbare Synthese aus billigen Precursoren zur Verfügung. Der Katalysator ist mehrfach rezyklierbar.

Die Reaktion kann ohne Lösungsmittel, also in Substanz, oder in einem Lösungsmittel durchgeführt werden.
Als Lösungsmittel sind alle organischen Lösungsmittel oder deren Mischungen mit Wasser geeignet, in denen der Katalysator stabil ist und die gegen den Katalysator stabil sind. Z.B. darf die Aktivkomponente (Heteropolyanion) nicht nennenswert vom Träger gelöst werden oder das Lösungsmittel (insbesondere Alkohole) unter den gewählten Reaktionsbedingungen nicht selbst oxidiert werden oder das Lösungsmittel mit dem Alkohol-Edukt (z.B. Säuren zu Estern) oder mit Reaktionsprodukten (z.B. Anhydride mit Wasser zur Säure) reagieren.
Geeignete Lösungsmittel sind z.B. Benzol, alkylierte Aromaten insbesondere Toluol, Xylole, chlorierte und fluorierte C₁-C₁₀-Alkane und Aromaten, Dichlorethan, Chlorbenzole insbesondere Monochlorbenzol, o- oder m-Dichlorbenzol, Benzonitril, Alkancarbonsäuren insbesondere Essigsäure, Alkylacetate, insbesondere Ethylacetat und Butylacetat, flüssige Alkane und Cycloalkane wie z.B. Dekan, Acetonitril, Benzonitril, DMF, Dimethylacetamid, Dimethylsulfoxid (DMSO), alkylierte Naphthaline, alkylierte Biphenyle, Dekalin, Tetralin, Diphenylmethan, Silikonöle, oder deren Gemische mit Wasser.

Für die Dimethylverbindung besonders geeignet sind Chlorbenzol, Dichlorbenzole, Ethylacetat oder Butylacetat.

Der bei dem erfindungsgemäßen Verfahren verwendete Katalysator ist aus der Literatur bekannt (Fujibayashi, Nakayama, Hamamoto, Sakaguchi, Nishiyama, Ishii, J. Mol. Catal. A 110 (1996) 105-117). Auf diese Literatur wird hiermit ausdrücklich Bezug genommen. Es handelt sich um einen NPVMo/C-Katalysator bzw. ein auf Aktivkohle geträgertes Ammoniummolybdatovanadophosphat als Aktivkomponente. Ganz allgemein besteht der Katalysator aus einem Heteropolyoxometallatanion enthaltend die Elemente P,V,Mo und einem Alkali, Erdalkali und/oder Ammonium als Gegenion, bevorzugt Ammonium, auf einem Aktivkohleträger. Bevorzugt sind 12-Metallophosphate.

### 1.) Hier wird die Oxidation von aktivierten Alkoholen mit benzylischen oder allylischen Protonen bei niedrigen Temperaturen < 120°C beschrieben.

Heteropolyanionen gehören zur Gruppe der Polyoxometallate und treten in einer fast unübersehbaren Vielfalt auf Die bekanntesten Vertreter weisen die sogenannte Keggin-Struktur auf, die sich dadurch auszeichnet, daß typischerweise ein Atom der 3., 4, oder 5. Hauptgruppe (z. B. B, Si, P, As, etc.) tetraedrisch von M₃O₁₀-Einheiten umgeben ist (M = Mo,W) die wiederum untereinander über Sauerstoffatome verknüpft sind. Die allgemeine Summenformel lautet H₃XM₁₂O₄₀. Daneben existieren noch eine Vielzahl an Defektstrukturen und größeren Aggregaten z. B. sog. Dawson-Typ Heteropolysäuren.
Die wolframhaltigen Heteropolysäuren zeichnen sich vor allem durch ihre große Säurestärke aus, während die molybdänhaltigen auch ausgeprägte Redoxeigenschaften aufweisen. Molybdän und Wolfram lassen sich untereinander gegenseitig und auch durch andere Metalle ersetzen, z. B. Nb, V. Dadurch, und durch die Wahl des geeigneten Heteroatoms, lassen sich die verschiedenen Eigenschaften der Heteropolysäuren gezielt beeinflussen. Darüber hinaus können weitere Modifikationen der Eigenschaften durch Substitution der Protonen durch Metallionen erreicht werden. Besonders geeignet für die vorliegende Erfindung sind Heteropolyphosphorsäuren des Molybdäns, in denen das Molybdän in hohem Maß durch Vanadium substituiert ist. Diese Substitution senkt den pKs-Wert und erhöht die Reduzierbarkeit der Heteropolysäure. Bis zu einem Substitutionsgrad von 3 entstehen bei der Synthese definierte Heteropolysäuren des Keggin-Typs. Bei dem hier vorliegenden Substitutionsgrad wird allerdings keine definierte Verbindung, sondern ein kompliziertes Gemisch aus Positions- und Substitutions- und Strukturisomeren des Molybdovanadophosphates erhalten.

Für die vorliegende Erfindung besonders geeignet ist ein Ammonium-Molybdatovanadophosphat, das aus NaVO₃ und Na₂MoO₄ durch Zugabe von H₃PO₄ und Eintrag der wässrigen Lösung in NH₄Cl-Lösung und Isolierung und Reinigung des Niederschlages erhalten wird. Diese Aktivkomponente wird anschließend durch Tränken auf den Aktivkohleträger aufgebracht. Aktivkohleträger sind bekannt als kostengünstige Träger für Edelmetalle und aufgrund ihrer großen inneren Oberfläche als Adsorbentien. Die innere Oberfläche ist im allgemeinen mit funktionellen Gruppen besetzt, die der Aktivkohle sowohl saure als auch basische Eigenschaften verleihen können. Manche Aktivkohlen enthalten einen Anteil an Heteroatomen (O, N, H), der mehr als 10 Gew.% betragen kann. Je nach Herstellung können Aktivkohlen mikroporös oder auch mesoporös sein.
Besonders geeignet für die vorliegende Erfindung sind chemisch aktivierte Aktivkohleträger, die sich durch einen hohen Anteil an großen Poren auszeichnen, und deren innere Oberfläche aus diesem Grund deutlich kleiner ist als 1400-1600 m²/g, die typischerweise von wasserdampfaktivierten, mikroporösen Aktivkohlen erreicht werden.
Bei den getesteten Aktivkohlen haben sich besonders solche Typen bewährt, die eine BET-Oberfläche im Bereich von 500 bis 1500 m²/g, bevorzugt 1000 bis 1400 m²/g haben.
Der pH-Wert der Aktivkohleträger kann zwischen 1 und 10 liegen, bevorzugt zwischen 2 und 6.
Der Träger kann mit weiteren (inerten) Komponenten, Bindern und Zusätzen abgemischt werden und in Form von Pellets, Kugeln, Tabletten, Ringen, Strängen, Sternen oder anderen Formkörpern oder als Splitt, Paste oder Pulver benutzt werden. Der Durchmesser bzw. die Länge und Dicke der Trägerformkörper liegt im allgemeinen bei 1 bis 10 mm. Für die Suspensionsfahrweise in der Flüssigphase sind aber keine Formkörper notwendig und es kann natürlich auch mit Pulvern, Pasten oder Splitt gearbeitet werden.

Der Katalysator kann weiterhin mit anderen Metallen und Nichtmetallen promotiert oder dotiert sein.

Die NPVMo-Beladung des Katalysators liegt i.a. im Bereich von 1 bis 25 Gew.-%, vorzugsweise im Bereich von 3 bis 15 Gew.%. Die notwendige Beladung kann in einem Schritt oder durch Mehrfachabscheidung aufgebracht werden.

Für die Trägerung des Heteropolyanions auf dem Aktivkohleträger können die üblichen Verfahren wie Tränken, Imprägnieren, Tauchen, Sprühimprägnieren etc. angewandt werden.

Das erfindungsgemäße Verfahren zeichnet sich insbesondere durch die damit erreichbaren hohen Umsätze bei gleichzeitig sehr guter Selektivität aus. So liegt die Selektivität der Reaktion nach 100 Stunden bei ≥ 90% bei einem Umsatz von > 90%.

Die folgenden Beispiele sollen die Erfindung erläutern.

### Beispiel 1: Herstellung des Katalysators

### 1) Präparation der NPVMo-Aktivkomponente

In einem 250 ml Mehrhalskolben werden 14.64 g Natriummetavanadat NaVO₃.H₂O vorgelegt, in 76 ml bidest. Wasser gelöst und gerührt. Es entsteht eine milchige Lösung.

16.4 g Natriummolybdat-2-hydrat Na₂MoO₄.2H₂O werden in 24 ml bidest. Wasser gelöst und dann zur NaVO₃-Lösung dazugegeben. Es wird für 10 min nachgerührt.

Über einen 50 ml Tropftrichter werden eine Lösung aus 15.2 g H₃PO₄ (85%) in 20 ml bidest. Wasser langsam dazugetropft. Es tritt sofort eine Gelbfärbung ein, die gegen Ende des Zutropfens in ein dunkles Rot übergeht.

Es wird 1 h bei 95°C nachgerührt und über Nacht stehengelassen.

Der Kolbeninhalt wird in 300 ml einer gesättigten Ammoniumchlorid-Lösung eingerührt. Es entsteht ein brauner Niederschlag. Dieser Niederschlag wird über eine Glasnutsche abfiltriert und dann 2x mit einer 0.25 molaren (50 ml) H₂SO₄ gereinigt. Der Niederschlag wird über Nacht im Trockenschrank getrocknet.

### 2.) Trägerung der NPVMo-Aktivkomponente auf Aktivkohle

Der in den Beispielen verwendete Aktivkohleträger hat eine BET-Oberfläche von 1300 m²/g und einen pH-Wert von 4
12 g der obigen Aktivmasse werden mit 400 ml dest. Wasser versetzt und 2 h bei Raumtemperatur gerührt. Diese Lösung wird dann abfiltriert, es entsteht eine klare rote Lösung. Zu dieser roten Lösung kommen 108 g Aktivkohle. Die Mischung wird 4 h bei Raumtemperatur gerührt, dann über eine Nutsche abgesaugt. Das schwarze Pulver wird im Trockenschrank getrocknet.

### Beispiel 2: Versuch im Laborautoklaven (Versuch 6_09)

In einem 100 ml Laborautoklaven mit Magnetrührer der Fa. Roth werden 0,38 g = 2,5 mmol 1,5-Dimethylbicyclo(3.2.1)octan-8-ol als Edukt in 10 ml Chlorbenzol als Lösungsmittel und 250 mg Katalysator vorgelegt (d.h. 90 ml Gasraumvolumen) und nach Erreichen der Reaktionstemperatur von 150°C werden 15 bar synthetische Luft aufgepreßt. Im Verlaufe der Reaktion wird aufgrund des Luftverbrauchs ein Druckabfall im Autoklaven beobachtet. Die Reaktionsdauer beträgt 15 h. Es wird keine Luft nachgepreßt, d.h. die Sauerstoffmenge ist limitiert. Bei 15 bar Luft = 3 bar O₂ und 150°C beträgt die vorgelegte Sauerstoffmenge ca. 7.7 mmol, d.h. es liegt ein ca. 3-facher molarer Sauerstoffüberschuß vor.

Die Analytik erfolgt auf einem HP 5890 GC mit Kapillarsäule Supelco 2-5358 (105m*0,53 mm, 3mm Film, isotherm 145°C). Die Analytik wird nur qualitativ durchgeführt, d.h. die im folgenden angegebenen Umsätze und Ausbeuten sind ohne Korrektur/Quantitative Eichung aus den integrierten Peakflächen berechnet.

Die Ergebnisse der katalytischen Austestung finden sich in Tabelle 1.

### Beipiele 3 bis 78; Weitere Versuche in Laborautoklaven

Die Autoklaven-Versuche werden in einem 100 ml Autoklaven der Fa. Roth oder in einem 200 ml Autoklaven mit Teflonauskleidung der Fa. Berkhoff durchgeführt. Beide Reaktoren sind mit einem Magnetrührer ausgestattet.

Der Versuchsablauf ist dergleiche wie in Beispiel 2, jedoch bei jeweils unterschiedlichen Reaktionsbedingungen, Konzentrationen, Lösungsmitteln und Verfahrensführungen, die zusammen mit den erhaltenen Ergebnissen aus der Tabelle 1 ersichtlich sind (Ausbeute, Umsatz und Selektivität in GC-Flächen-%).

Bei einer Absenkung der Reaktionstemperatur unter 150°C nimmt die Reaktionsgeschwindigkeit drastisch ab.
Höhere Reaktionstemperaturen im Bereich 180 - 220°C erlauben eine deutliche Steigerung der Reaktionsgeschwindigkeit und damit Absenkung der Reaktionsdauer ohne Selektivitätseinbuße. Diese Versuche bei erhöhter Temperatur sind noch einmal extra in der Tabelle 5 aufgeführt.

Die Versuche zur Variation von Eduktmenge und Katalysatormenge sind noch einmal extra in der Tabelle 3 und Tabelle 4 zusammengefaßt.

Verfahrensvarianten, die einen lokalen Sauerstoffunterschuß am Aktivzentrum des Katalysators aufrechterhalten, erlauben noch Selektivitätsverbesserungen. So kann der Druck auf <10 bar abgesenkt werden.
Als vorteilhaft hat sich eine kontinuierliche oder auch diskontinuierliche Sauerstoffnachführung bei niedrigen Drucken oder sogar drucklos erwiesen.

### Beispiele 79 - 83: Drucklose Variante in einer Labor-Rückflußapparatur

Die drucklosen Versuche wurden in einer Rückflußapparatur bestehend aus Mehrhalskolben mit Magnetrührer, Gaseinleitungsrohr, Innenthermometer und Intensivkühler bei kontinuierlicher Sauerstoff-/Luftzufuhr unter Normaldruck durchgeführt. Die Steuerung der Gaseinleitung erfolgte über einen MFC bei einem Gasfluß von 2,5 l/h. Das Lösungsmittelvolumen betrug 50 ml.

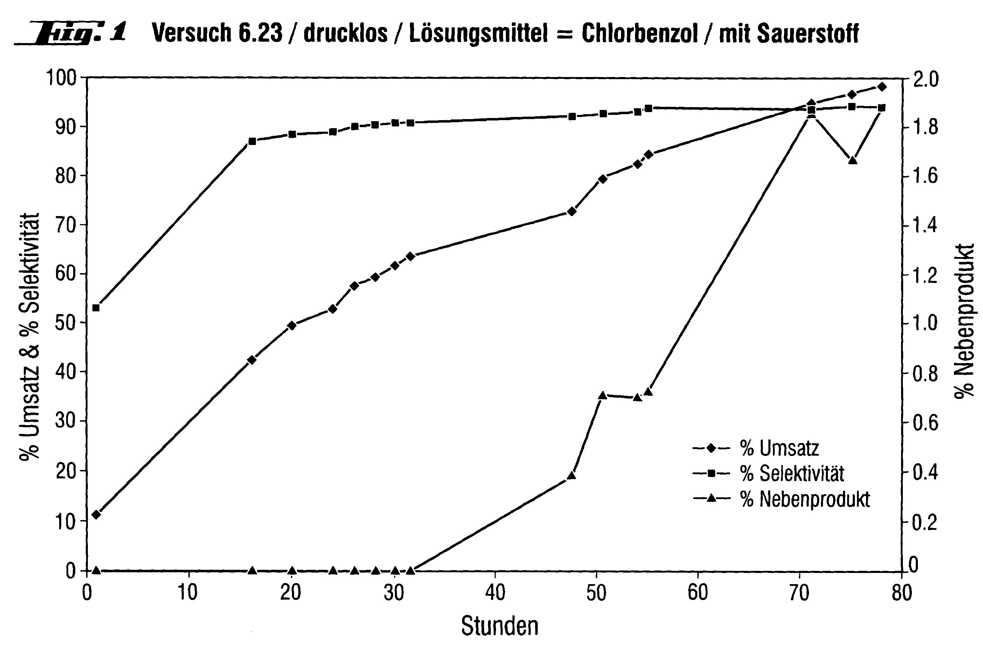

Die Ergebnisse der katalytischen Austestung sind den Figuren 1-5 zu entnehmen.

### Reiner Sauerstoff als Oxidationsmittel:

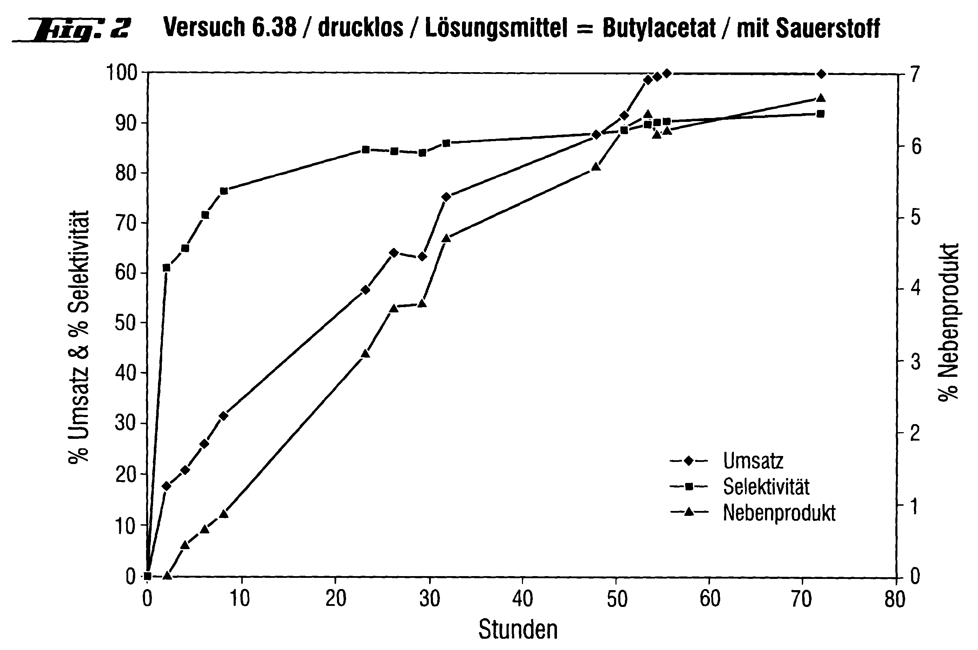

Die Figuren 1 und 2 zeigen den Zeitverlauf der Oxidation in Chlorbenzol bzw. Butylacetat als Lösungsmittel. Man erkennt, daß in Chlorbenzol in 70 h Vollumsatz erreicht wird, während die Selektivität ca. 90% beträgt. Das einzige nennenswerte GC-Nebenprodukt (RT=31.3 in Chlorbenzol) tritt erst ab 30 h auf und wächst dann kontinuierlich bis auf ca. 2% an.
Aus dem zeitlichen Konzentrationsverlauf dieses Nebenproduktes bieten sich für die technische Realisierung zwei Verfahrensvarianten an, nämlich hochselektiv bei Teilumsatz oder Vollumsatz unter Umgehung der Eduktabtrennung aus dem Reaktionsgemisch.

In Butylacetat wird Vollumsatz schon nach 50 h erreicht. Das Nebenprodukt wird auch schon zu Reaktionsbeginn gefunden und wächst im weiteren Reaktionsverlauf etwa linear bis auf ca. 6% an. Da bei bereits 100%-igem Umsatz immer noch weiteres Nebenprodukt gebildet wird, kann gefolgert werden, daß es über eine Folgereaktion aus bereits gebildetem Produkt entsteht (vermutlich Weiteroxidation des Ketons mit Ringöffnung des Bicyclus). Ebenso folgt daraus für die technische Reaktionsführung, daß die Reaktion zum richtigen Zeitpunkt (kurz nach Erreichen von Vollumsatz) abgebrochen werden muß, um unnötige Selektivitätsverluste zu vemeiden.

### Luftoxidation:

Figur 3 zeigt den Zeitverlauf der drucklosen Luftoxidation (Gasfluß 2.5 l/h Luft) in Chlorbenzol.
Es wird in 70 h bei 100% Umsatz eine Selektivität >95% erreicht.
Figur 4 zeigt den Zeitverlauf der drucklosen Luftoxidation (Gasfluß 2.5 l/h Luft) in Butylacetat.
Es wird in 80 h bei 100% Umsatz eine Selektivität >90% erreicht.

Die Selektivität kann durch Verwendung von Luft anstelle von reinem O₂ somit gesteigert werden.

Figur 5 zeigt den Zeitverlauf der drucklosen Luftoxidation (Gasfluß 2.5 l/h Luft) in Chlorbenzol mit der 3-fachen Katalysator- und Eduktmenge. Nach 55 h wird Vollumsatz bei einer Selektivität >95% erreicht.
Die Verdopplung und Verdreifachung der Edukt- und Alkoholmenge sind damit ohne weiteres noch möglich. Höhere Konzentrationen sind wünschenwert, da die Stoffaufarbeitung vereinfacht wird (Versuch 6.80).

### Beispiel 84: Technikumsversuche im 1 l Autoklaven (Versuche zur Maßstabsvergrößerung)

Es wurden zwei Versuche (mit jeweils verschiedenen Edukt/Katalysator-Konzentrationen) im 1-Liter-Autoklaven in einem Hochdrucktechnikum durchgeführt. Dabei wurden jeweils 400 g Chlorbenzol vorgelegt und
für Versuch 6.67: 14 g Kat + 15.2 g Alkohol
für Versuch 6.78: 28 g Kat + 30.4 g Alkohol (Verdopplung von Kat./Alkoholmenge) bei 150°C und 2.5 bar Magerluft (10% O₂) bei einem Gasfluß von 50 l/h eingesetzt, wobei mit einer Drehzahl von 600 U/min mechanisch gerührt wurde. Das Produkt wurde nicht isoliert, sondern nur GC-Analysen der Reaktionsmischung vorgenommen.

Es werden noch bessere Ergebnisse erzielt als bei den Laborautoklav-Versuchen. Der Versuch 6.67 (Einsatz der Standardkonzentrationen von Kat./Alkohol) ergibt Vollumsatz bei 91% Selektivität (auf Basis GC-Flächen-%) nach nur 24 h, während in der drucklos betriebenen Laborapparatur noch 70 h notwendig waren, d.h. die Reaktionsdauer kann durch Optimierung der Durchmischung (Gasfluß, Rührerdrehzahl) noch drastisch gesenkt werden.
Die gleichzeitige Verdopplung von Katalysator- und Alkohol-Konzentation führt ohne Selektivitätsverlust zu einer weiteren Absenkung der Reaktionsdauer auf 15 h.

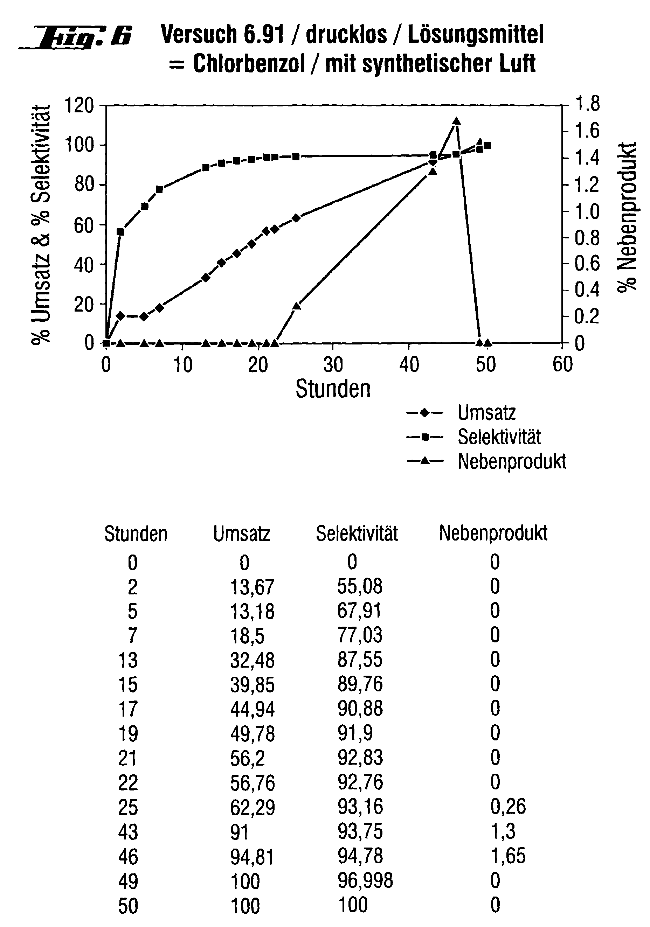

Die Tabellen 2 und 3 fassen die Versuche 6.67 und 6.78 noch einmal zusammen. Beispiel 85: Versuch zur Maßstabsvergrößerung im 4-Liter-Rührkolben Figur 6 zeigt den Zeitverlauf der Luftoxidation in einer drucklos betriebenen und mechanisch gerührten Rückflußapparatur mit 4 Liter-Kolben (Versuch 6.91, 1 kg Chlorbenzol, 70 g Katalysator, 76 g Alkohol, 150°C, Gasfluß 7.5 l/h synthetische Luft).
Es wird 100% Umsatz und >95% Selektivität nach 50 h erreicht.

### Tabelle 1: Laborautoklavenversuche

Ergebnisse der katalytischen Oxidation von 1,5-Dimethylbicyclo(3.2.1.)octan-8-ol zum Keton
   (Ausbeuten und Selektivitäten in GC-Flächen-%)

### Figuren 1 - 5: Drucklose Variante in einer Labor-Rückflußapparatur

Ergebnisse der katalytischen Oxidation von 1,5-Dimethylbicyclo(3.2.1.)octan-8-ol zum Keton
   (Ausbeuten und Selektivitäten in GC-Flächen-%)
   Figur 1: Reiner Sauerstoff, Chlorbenzol
   Figur 2: Reiner Sauerstoff, Butylacetat

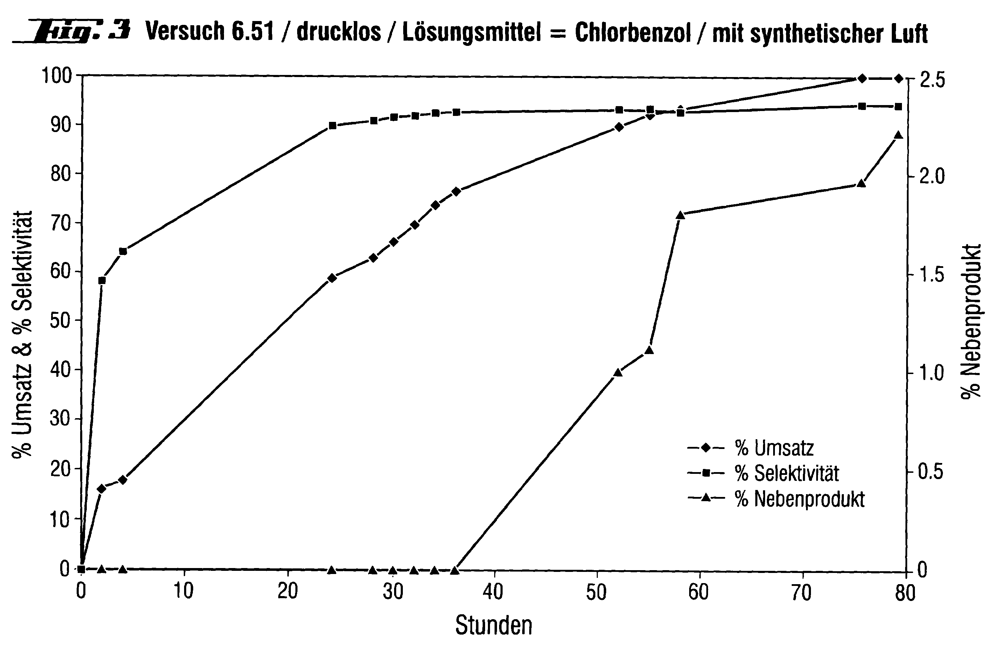

   Figur 3: Luft, Chlorbenzol

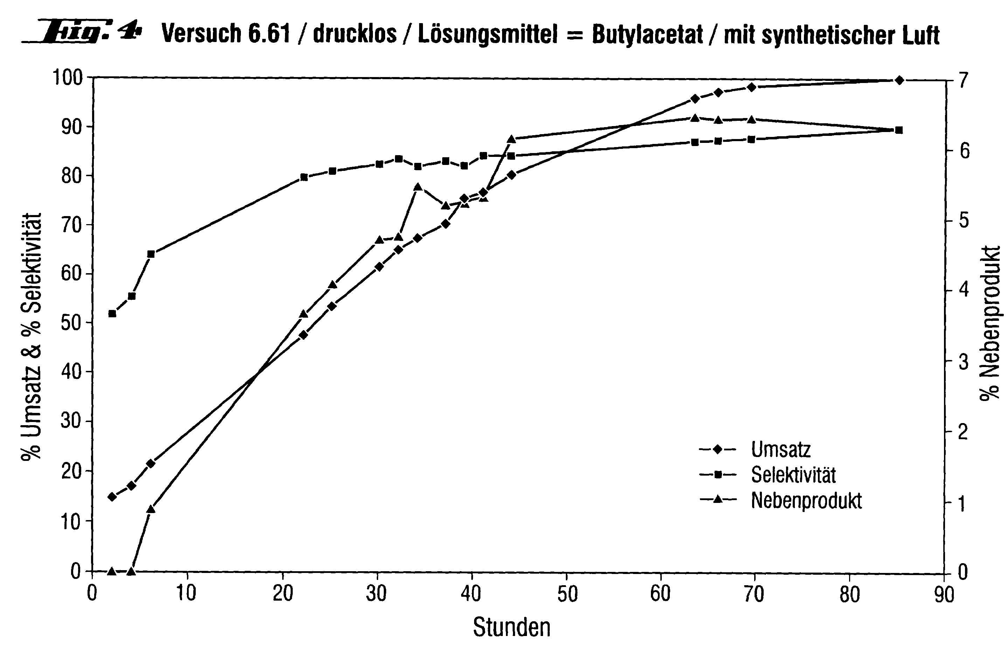

   Figur 4: Luft, Butylacetat

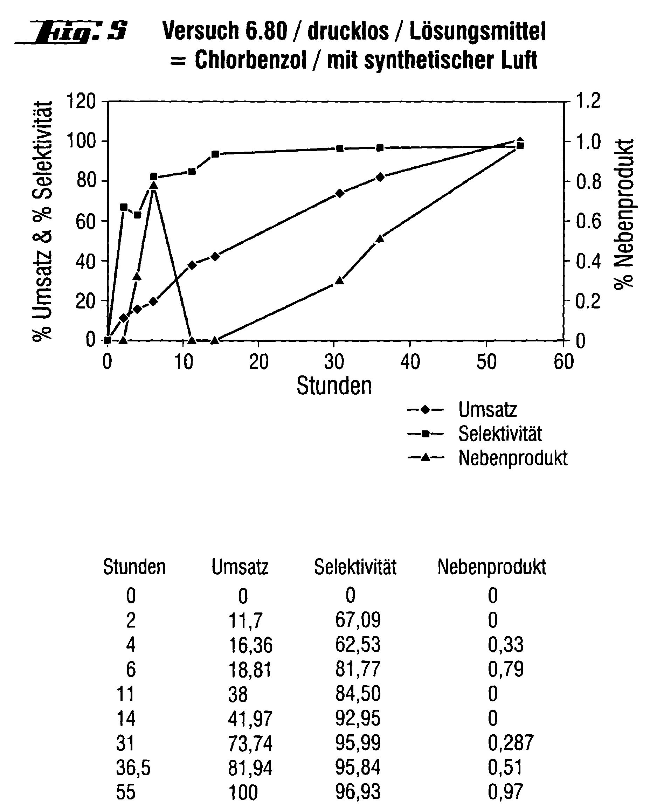

   Figur 5: Luft, Chlorbenzol, 3-fache Katalysator- und Eduktmenge
   Figur 6: Maßstabsvergrößerung im 4-Liter-Rührkolben (drucklos, Chlorbenzol, Luft)
Ergebnisse der katalytischen Oxidation von 1,5-Dimethylbicyclo(3.2.1.)octan-8-ol zum Keton

### Tabellen 2 und 3: Technikumsversuche mit Magerluft im 1 l Autoklaven

Ergebnisse der katalytischen Oxidation von 1,5-Dimethylbicyclo(3.2.1.)octan-8-ol zum Keton
   Tabelle 2: Versuch 6.67
   Tabelle 3: Versuch 6.78

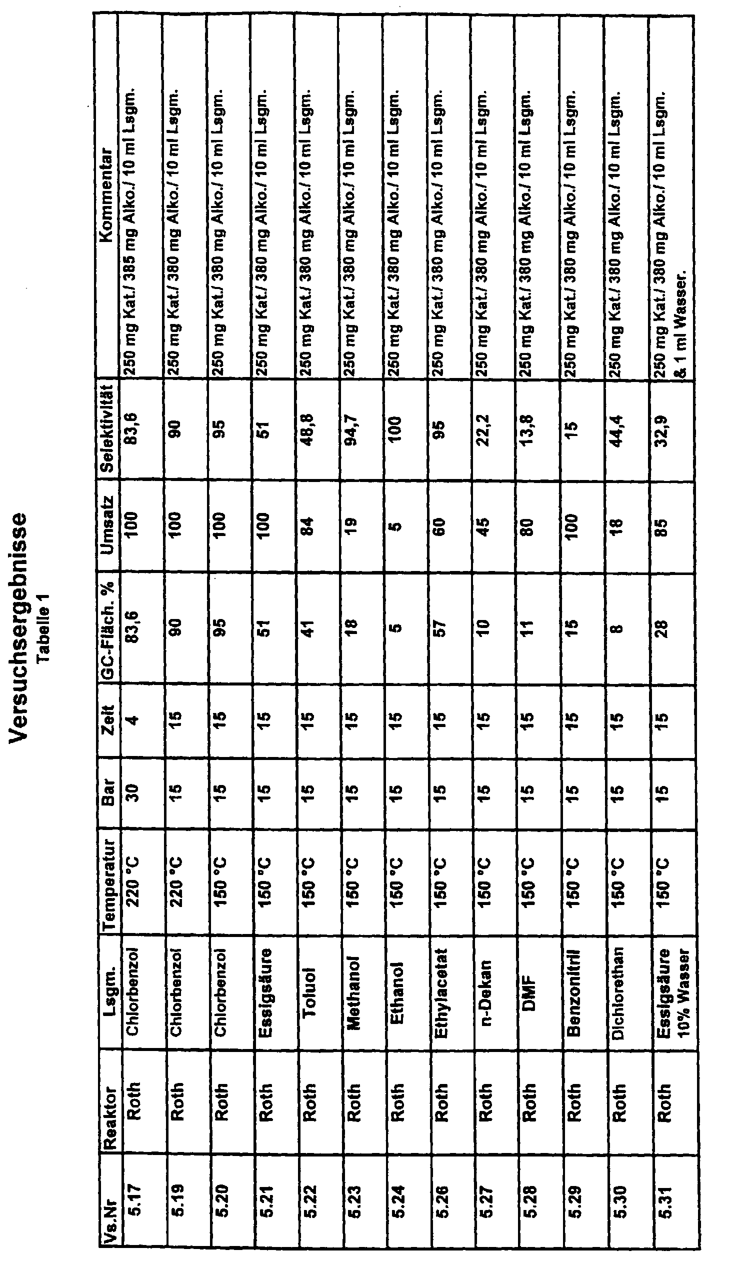

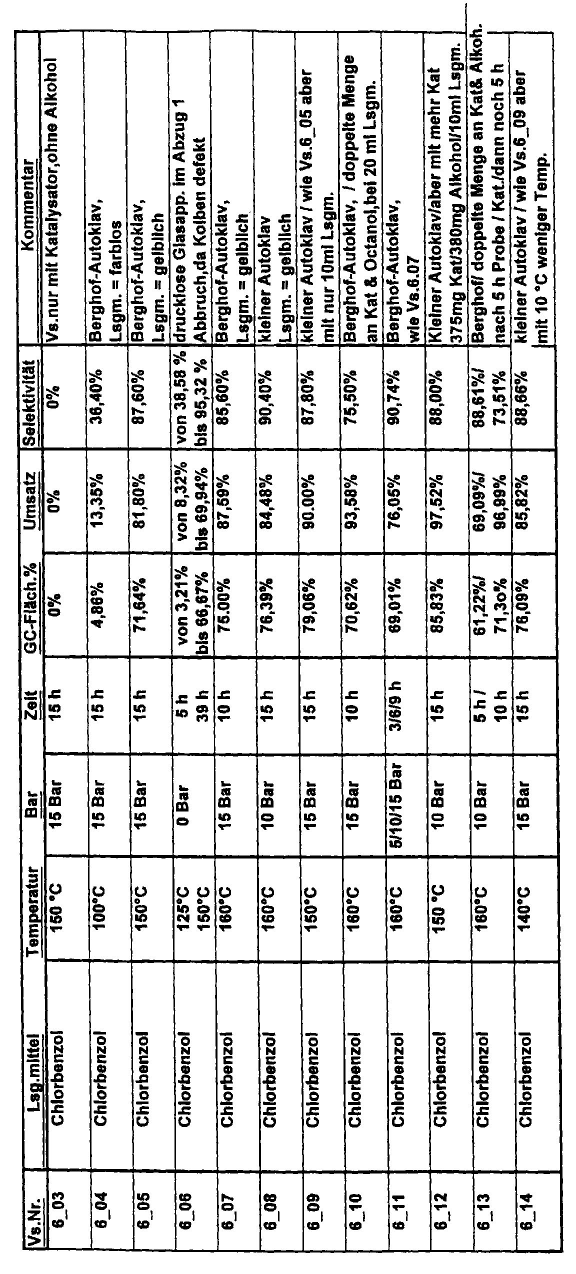

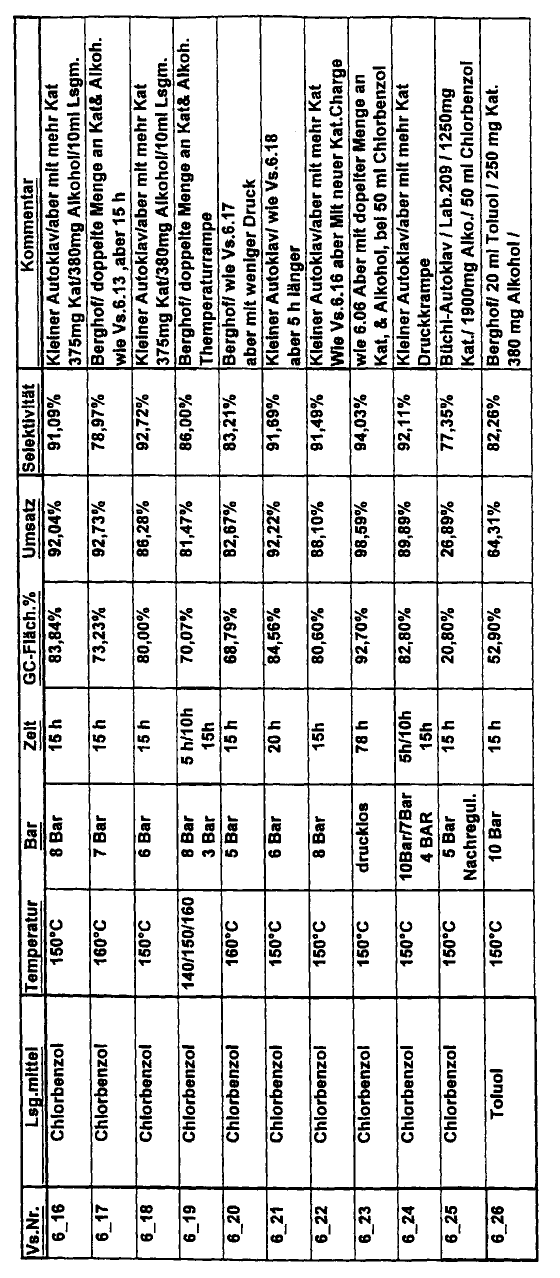

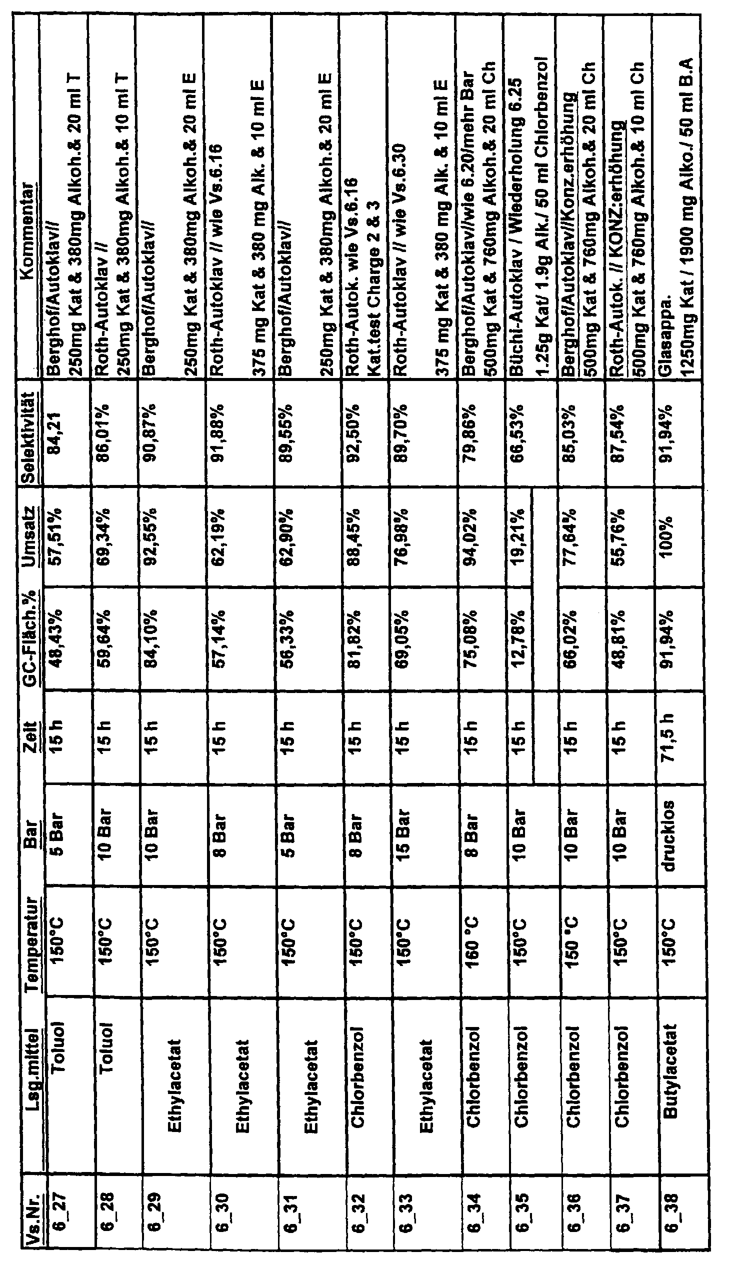

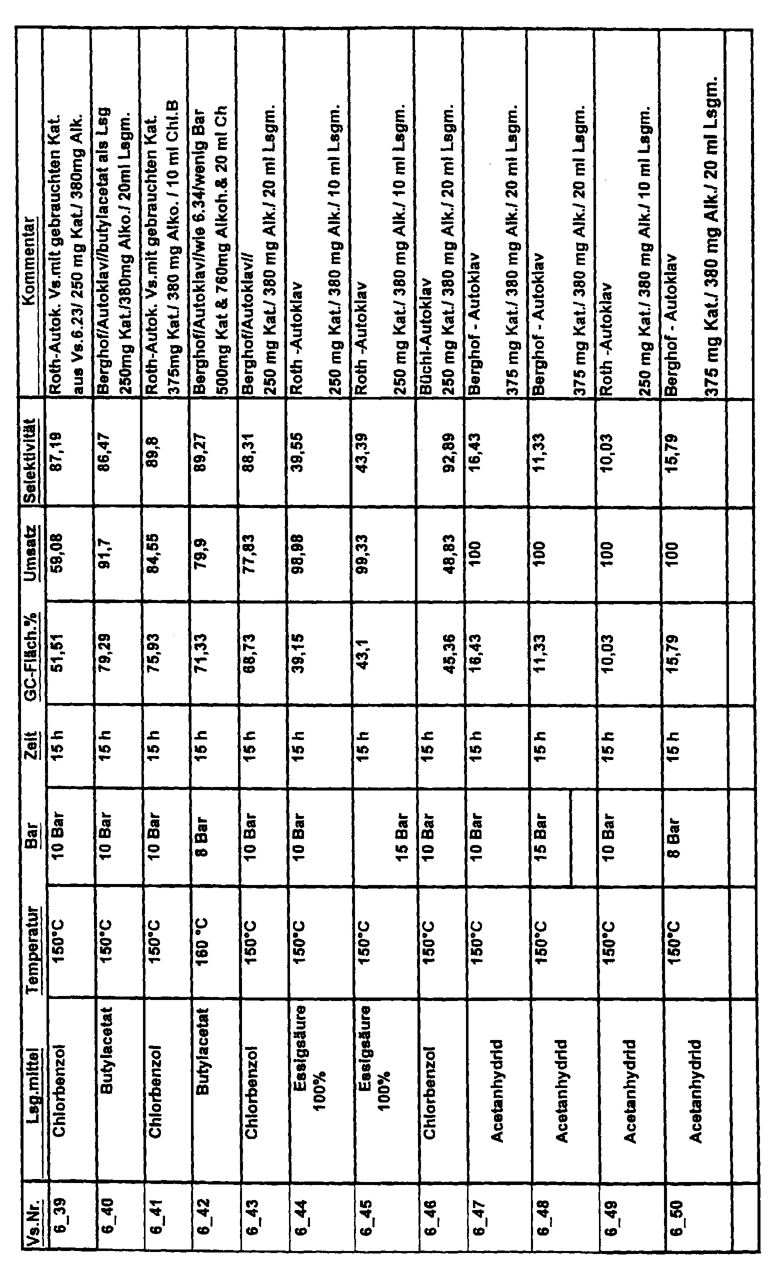

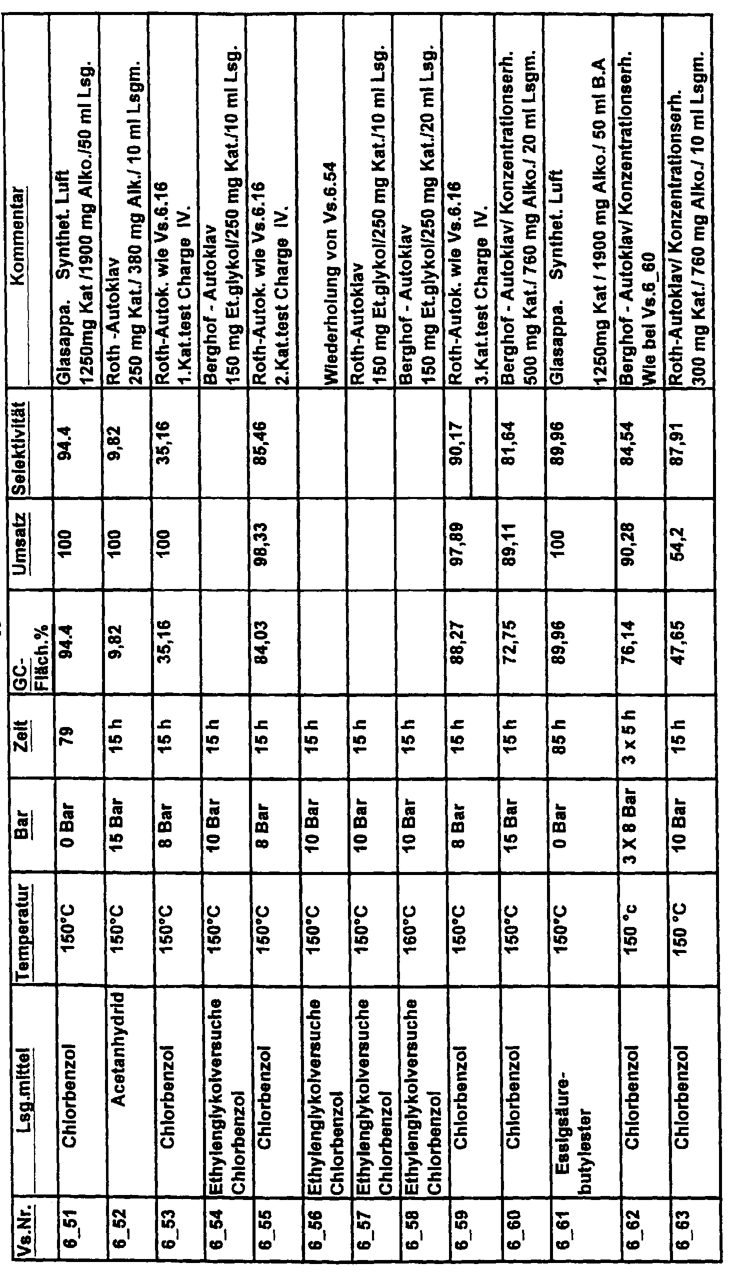

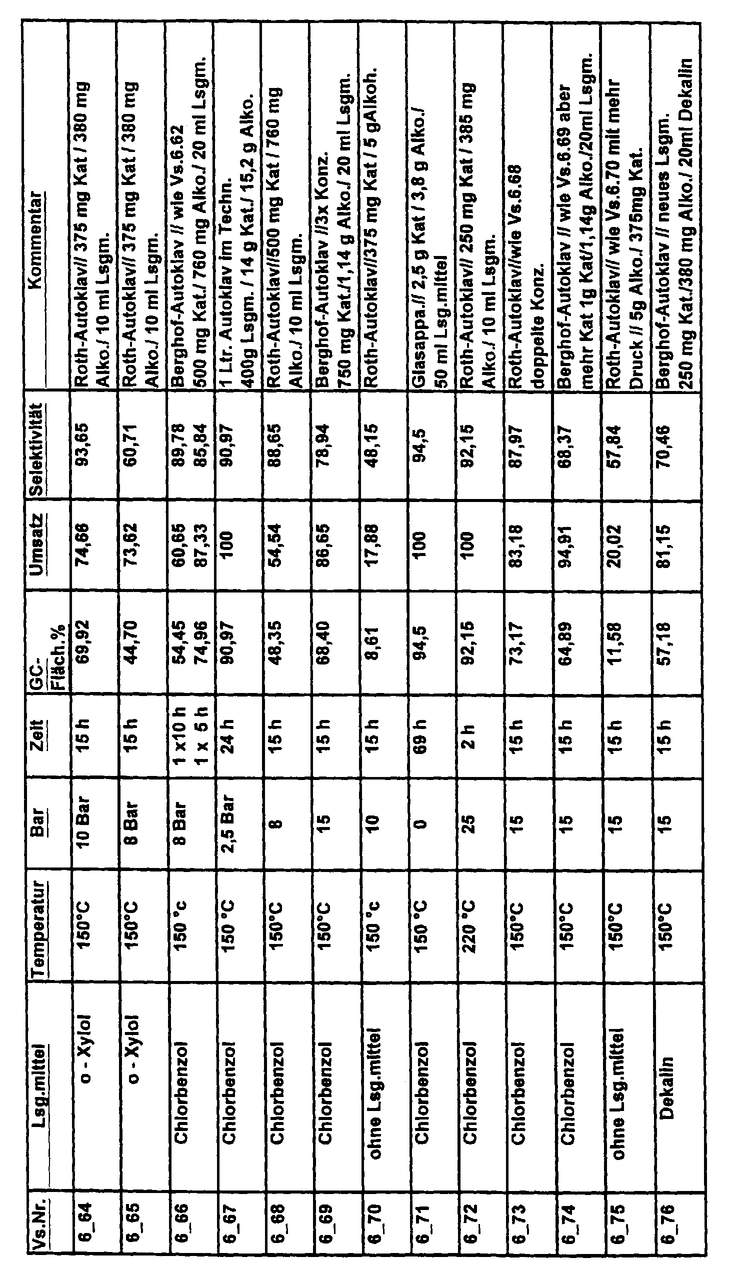

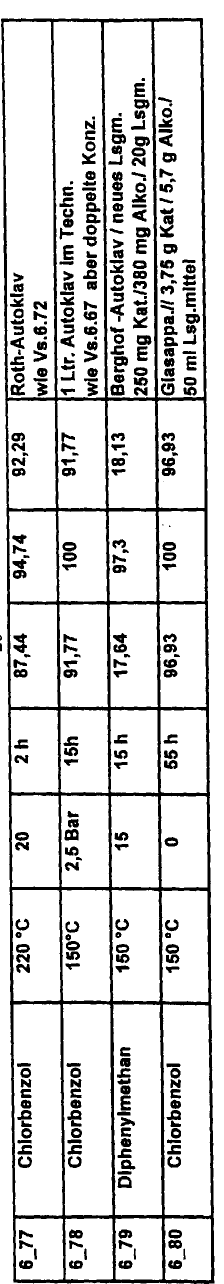

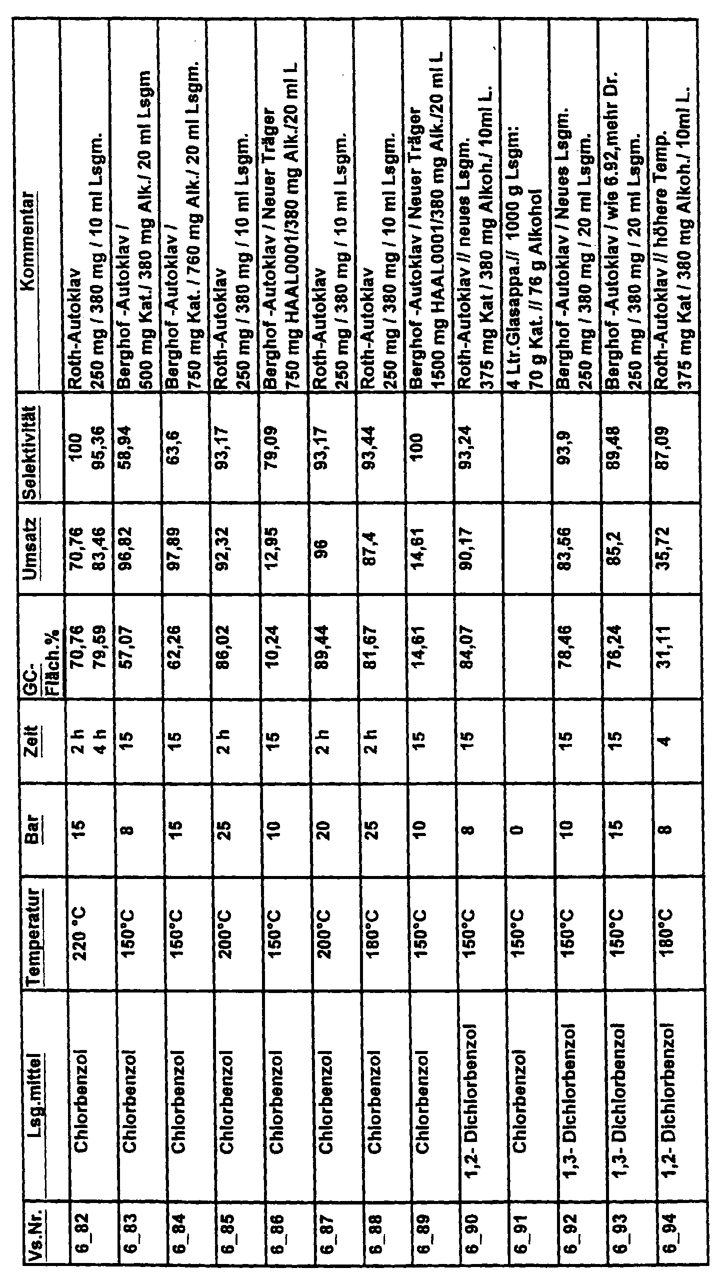

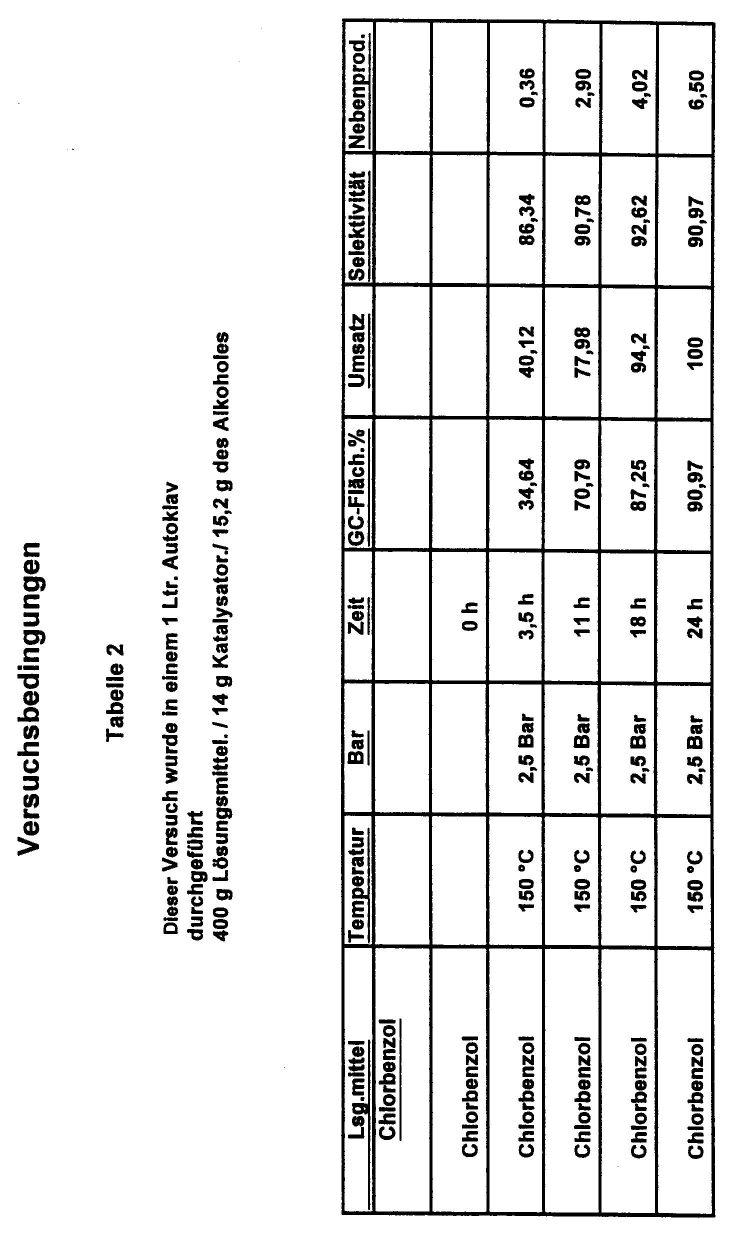

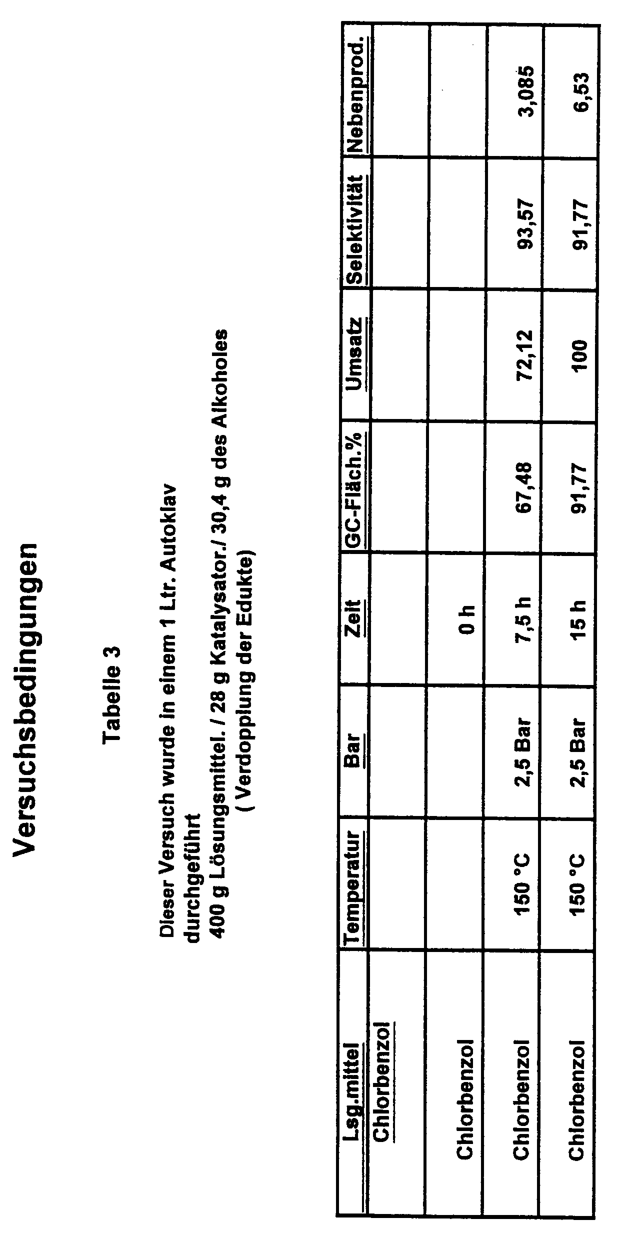

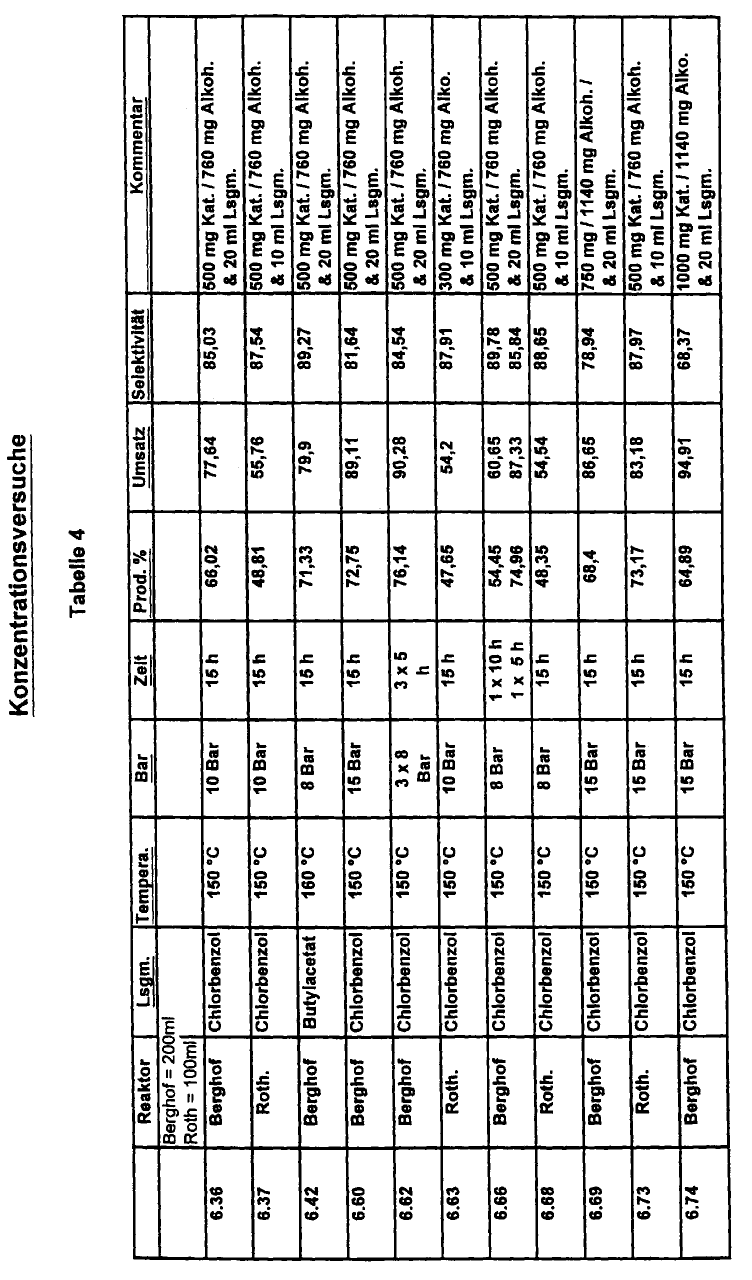

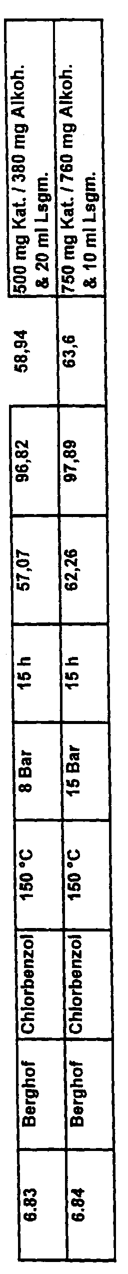

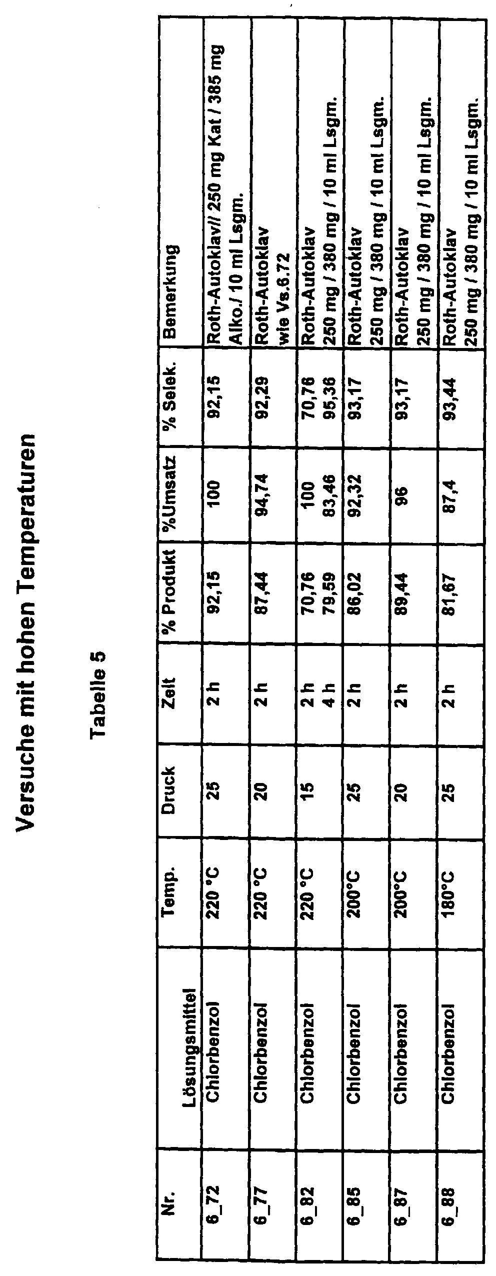

## Patentansprüche

1. Verfahren zur Herstellung von Bicyclooctanon durch Umsetzung von Bicyclooctanol- mit
a. einem auf einem Aktivkohleträger aufgebrachten Katalysator enthaltend Heteropolyoxometallatanionen von Vanadium, Molybdän und Phosphor sowie ein entsprechendes Alkali-, Erdalkali- und/oder Ammonium-Gegenion und
b. einem gasförmigen, sauerstoffhaltigen Oxidationsmittel

2. Verfahren nach Anspruch 1, wobei als Oxidationsmittel Luft oder Magerluft verwendet wird .

3. Verfahren nach Anspruch 1 oder 2, wobei das Verfahren bei einem Druck von 1 bis 30 bar durchgeführt wird.

4. Verfahren nach mindestens einem der Ansprüche 1 bis 3, wobei das Verfahren bei Normaldruck durchgeführt wird.

5. Verfahren nach mindestens einem der Ansprüche 1 bis 4, wobei die Temperatur in einem Bereich von 100 bis 300°C liegt.

6. Verfahren nach mindestens einem der Ansprüche 1 bis 5, wobei die Reaktionsmischung gerührt wird.

7. Verfahren nach mindestens einem der Ansprüche 1 bis 6, wobei Dialkylbicyclo(3.2.1.)octan-8-ol zu 1,5-Dialkylbicyclo(3.2.1.)octan-8-on umgesetzt wird.

8. Verfahren nach 7, wobei Alkyl unabhängig voneinander für H oder einen verzweigten oder unverzweigten Methyl, Ethyl, Propyl, Butyl, Pentyl, Hexyl oder Cyclohexylrest steht.

9. Verfahren nach mindestens einem der Ansprüche 1 bis 8 wobei das Verfahren in einem Lösungsmittel durchgeführt wird.

10. Verfahren nach mindestens einem der Ansprüche 1 bis 9, wobei als Lösungsmittel Chlorbenzol, Dichlorbenzole oder Butylacetat verwendet wird.

11. Verfahren nach mindestens einem der Ansprüche 1 bis 10, wobei als Katalysator ein auf Aktivkohle geträgertes Ammoniummolybdatovanadophosphat verwendet wird.

12. Verfahren nach mindestens einem der Ansprüche 1 bis 11, wobei der Aktivkohleträger eine BET-Oberfläche im Bereich von 500 bis 1500 m²/g hat.

13. Verfahren nach mindestens einem der Ansprüche 1 bis 12, wobei die Selektivität nach 100 Stunden bei ≥ 90% bei einem Umsatz von >90% liegt.
